## Europäisches Patentamt
## European Patent Office
## Office européen des brevets

(11) Numéro de publication: **0 022 792**
**B1**

(12) **FASCICULE DE BREVET EUROPEEN**

(45) Date de publication du fascicule du brevet: **09.05.84**

(51) Int. Cl.³: **A 61 F 13/16,**
**A 61 L 15/00, A 41 B 13/02**

(21) Numéro de dépôt: **79901666.2**

(22) Date de dépôt: **05.12.79**

(86) Numéro de dépôt international:
**PCT/FR79/00120**

(87) Numéro de publication internationale:
**WO 80/01455 24.07.80 Gazette 80/17**

(54) PROCEDE D'INSERTION DE PRODUITS SUPER-ABSORBANTS DANS UNE STRUCTURE FIBREUSE.

(30) Priorité: **12.01.79 FR 7900717**

(43) Date de publication de la demande:
**28.01.81 Bulletin 81/4**

(45) Mention de la délivrance du brevet:
**09.05.84 Bulletin 84/19**

(84) Etats contractants désignés:
**AT DE GB LU NL SE**

(56) Documents cités:
**FR - A - 2 265 542**
**FR - A - 2 374 891**
**GB - A - 1 193 433**
**US - A - 2 788 003**
**US - A - 3 837 343**
**US - A - 3 903 889**
**US - A - 4 055 180**

(73) Titulaire: **BEGHIN-SAY SOCIETE ANONYME**
**F-59239 Thumeries (FR)**

(72) Inventeur: **HOLVOET, Marcel**
**Amfreville-sur-Iton**
**27400 Louvier (FR)**
Inventeur: **PIGNEUL, Raymond**
**2 Rue des Vosges**
**68329 Durrenentzen (FR)**

Courier Press, Leamington Spa, England.

## Description

La présente invention se rapporte à un procédé d'insertion de produits pulvérulents dits "super-absorbants" dans un matelas de fibres. L'invention concerne également les structures fibreuses absorbantes ainsi obtenues.

Technique anterieure

Les produits dits "super-absorbants" sont des composés qui gonflent en présence du liquid à absorber, mais qui ne se solubilisent pas: le liquide est alors absorbé de façon quasi-irréversible. Ce sont principalement des éthers cellulosiques, des alginates ou des polyacrylates modifiés.

Leur mise en oeuvre a fait l'objet de nombreuses études, et l'on connaît ainsi de nombreuses méthodes pour les insérer dans un support.

Selon la plupart des techniques connues, le produit super-absorbant est fixé à la surface du supprot ou bien incorporé dans la masse dudit support, les termes "fixé" ou "incorporé" signifiant qu'il existe une liaison rigide entre le produit généralement én poudre et le support.

Cette liaison rigide est obtenue par la présence d'un produit additif responsable de l'adhésion.

Cet additif, selon une première série de méthodes connues, peut être de l'eau: on parle alors de fixation du super-absorbant par mouillage.

Ainsi, selon la méthode décrite dans FR—A—2.319.434, le support est mouillé, puis les particules d'un éther cellulosique modifié sont déposées sur le support. Les particules gonflent et adhèrent au support, puis le liquide est éliminé par séchage. En variante, un procédé similaire est décrit dans le brevet américain 3.919.042, qui consiste à pulvériser par voie électrostatique des particules d'amidon sur une feuille de fibres cellulosiques contenant au moins 25% d'eau.

Selon d'autres méthodes, au contraire des précédentes, c'est le produit "super-absorbant" qui est humidifié. Par exemple, la méthode décrite dans FR—A—2.122.432 consiste à appliquer sur un support cellulosique un gel gonflé d'eau (polyacrylamide réticulé), puis à sécher le laminé obtenu. Ou encore, celle décrite dans le brevet allemand 489.308 selon laquelle on humidfie la couche de poudre d'amidon (ou de dextrine ou de gélatine) pour la fixer.

D'autres méthodes encore recourent à l'adjonction d'eau, comme agent de fixation de super-absorbant, à la fois sur le support et sur le produit absorbant. Ainsi, selon la technique décrite dans le brevet anglais 1.354.406, des feuilles d'ouate de cellulose, imprégnées ou non d'un composé cationique sont recouvertes d'une poudre d'un polymère hydro-absorbant, empilées et soumises à un traitement à la vapeur d'eau.

L'ensemble constitué par le support fibreux et la poudre absorbante est également humidifié par traitement à la vapeur d'eau selon l'objet du FR—A—2 066 324.

Les techniques de fixation par mouillage présentent, en général, l'avantage d'être peu coûteuses et simples, si l'on excepte pour certaines méthodes décrites plus haut l'opération de séchage. Mais elles présentent l'inconvénient de faire gonfler les particules absorbantes avant séchage. Ce qui se traduit par une perte d'efficacité des particules absorbantes, leur état de surface étant modifié.

En outre, plusieurs particules peuvent adhérer entre elles, ce qui conduit à la formation d'un film dans le cas, le plus probable, du dépôt d'une monocouche. Pour éviter la "gélification partielle" inévitable dans les procédés de fixation par mouillage, d'autres techniques de fixation recourent à l'utilisation d'un liant, autre que l'eau.

Ainsi, selon le brevet américain 3.903.889, on recouvre la surface de la structure fibreuse d'une couche de matière adhésive, ensuite on "saupoudre" des particules du produit absorbant les liquides sur ladite couche de matière adhésive.

Selon un autre exemple constitué par la demande de brevet FR—A—2 402 474 au nom de la demanderesse, l'ordre des étapes est inversé; en effet, le liant, qui est de préférence une solution aqueuse de poly-alcoolvinylique est déposé sur la surface du composite obtenu après pulvérisation ou transfert du produit super-absorbant sur le support.

Dans une technique plus-élaborée faisant appel à l'utilisation d'un liant autre que l'eau, le liant est un adhésif thermoplastique dispersé de façon intermittente à la surface supérieure d'une structure fibreuse.

Ainsi, le brevet américain No. 2.788.003 décrit un produit absorbant à jeter comprenant une nappe de fluff, insérée entre une feuille supérieure perméabie et une feuille inférieure imperméable, la feuille perméable étant revêtue de l'adhésif thermoplastique. La structure est soumise à un calandrage à chaud qui, outre son action de cohésion du fluff, a pour effet de faire fondre l'adhésif pouvant se trouver sur les lignes de matriçage, assurant ainsi la liaison en certains endroits entre la feuille supérieure et la couche médiane de fluff 3.

Tous les procédées de fixation du prouduit absorbant les liquides par un adhésif autre que l'eau présentent aussi l'avantage d'être simples et peu coûteux et l'inconvénient d'affaiblir la capacité d'absorption du produit absorbant par la présence de l'adhésif sur une partie de la surface des grains.

Pour cette raison on a cherché à utiliser une structure fibreuse contenant la poudre super-absorbante sans faire appel à un liant, de quelque nature que ce soit.

Le brevet anglais 1.193.433 divulgue une structure fibreuse enfermant un superabsorbant sous forme de poudre ou de granulé. Une telle structure en sandwich est réalisée par dépôt de la poudre sur un matelas absorbant, puis recouvrement par une nappe de fibres et compression par des rouleaux. Enfin, la structure ainsi obtenue est découpée pour former des éléments tels que pansements, protège-slips...

Malgré l'absence de liant, les articles obtenus selon ce procédé présentent parfois une diminution de leur pouvoir absorbant en certains endroits, en raison du manque d'homogénéité de leur structure interne. En effet, les granulés ou grains de poudre ne sont par toujours bien répartis, ils peuvent glisser, s'accumuler en certains points, créant ainsi des amas en certains endroits et des vides à d'autres responsables de la baisse du pouvoir absorbant.

Pour remédier à cet inconvénient on a cherché à maintenir la poudre en créant des points ou lignes de cohésion entre couche inférieure et couche supérieure entourant la poudre.

Le brevet américain 4.055.180 divulgue une structure fibreuse stratifiée destinée notamment aux serviettes périodiques et aux couches pour bébés, composée d'un matelas fibreux enveloppé d'une feuille de tissue-ouate sur laquelle est déposée sans être fixée une composition hydrocolloïdale sous forme d'une poudre. Le composite obtenu est revêtu d'une feuille de polyéthylène dans laquelle sont conformées de multiples cavités ou poches de forme semi-sphérique, par exemple, qui forment autant d'espaces clos pour l'expansion du gel. Dans cette réalisation, la poudre n'est pas "fixée", c'est-à-dire non collée au support. Au contraire dans chaque cavité formée, une quantité délimitée de poudre est simplement enfermée. Pour que le liquide à absorber puisse atteindre la poudre absorbante, la feuille de polyéthylène est percée de très nombreux petits trous.

Une telle structure ne remédie qu'en partie aux inconvénients précités du collage par liant ou du mouillage de la poudre et/ou du support.

En effet, on sait que le produit super-absorbant retient les liquides en augmentant son volume de façon très importante. Compte-tenu du volume forcément limité des poches préformées, la quantité de produit absorbant qui peut être introduite dans chacune d'elles reste elle-aussi limitée. Si cette quantité est trop importante, les parois des poches formées dans la feuille de polyéthylène constituent un obstacle à l'expansion du gel et, par conséquent, diminuent la capacité d'absorption de la structure.

D'autre part, la feuille de polyéthylène est préformée, ce qui rend beaucoup plus difficile la mise en oeuvre du procédé d'obtention du stratifiée. Enfin, l'écoulement du liquide par de nombreux petits trous de la feuille de polyéthylène qui peuvent s'obstruer facilement est très précaire.

Un connaît également une réalisation de la National Starch Company selon laquelle on dispose une grille thermoplastique entre deux feuilles de tissue-ouate, la poudre de super-absorbant étant simplement déposée à l'intérieure des mailles de la grille, et les deux plis de tissue-ouate étant maintenus par thermoliage sur la grille intermédiaire. Selon ce procédé, on évite le pré-formage d'une multitude de poches.

Mais de la même façon que pour le brevet US 4.055.180 déjà cité, la quantité de poudre de "super-absorbant" qui peut être disposée entre les plis de ouate est limitée. Dans cette réalisation, elle est limitée par la relative inextensibilité du pli de tissue-ouate.

Ainsi, bien que la poudre ne soit plus fixée, l'obstacle principal à l'expansion du gel réside dans les parois non élastiques de la poche contenant la poudre.

Expose de l'invention

Le but de la présente invention est de remédier à tous les inconvénients précités en réalisant un nouveau procédé d'insertion de poudre de produit super-absorbant dans un matelas de fibres, permettant l'insertion de doses importantes dudit produit qui joue pleinement son rôle d'absorbant, et cela d'une manière simple, continue, et donc peu onéreuse.

Pour atteindre ce but, on procède de la manière suivante:

— on dépose la poudre de produits super-absorbants en au moins une bande dont la largeur est fonction du produit à réaliser, sur un matelas fibreux.
— on recouvre l'ensemble d'une nappe de fibres;
— on lie ladite nappe de fibres uniquement par matriçage à froid audit matelas fibreux selon des lignes et/ou des points, de manière à définir entre ledit matelas et ladite nappe des espaces dans lesquels une certaine quantité de poudre de produit super-absorbant est emprisonnée, les grains etant ainsi libres dans les espaces expansibles ainsi constitués.

Selon le mode de réalisation de l'invention, le matelas fibreux et ladite nappe fibreuse sont tous deux des nappes de fluff de faible épaisseur. La nappe de fluff de recouvrement est alors avantageusement de largeur inférieure à celle sur laquelle est déposée la poudre, de manière à ne recouvrir que la zone saupoudrée. L'ensemble est matricé à froid à l'aide d'un cylindre dont la gravure forme un dessin réticulé des lignes de cohésion chaque maille emprisonnant sans liaison rigide une certaine quantité de poudre de produit super-absorbant.

Il est possible d'utiliser une telle structure fibreuse renfermant un produit super-absorbant en poudre dans un grande nombre d'articles absorbants, tels que couches, serviettes périodiques, alèzes etc.

La description ci-après d'exemples non limitatifs de réalisation de l'invention est complétée par la descriptiontion d'un exemple de serviettes périodiques dont la partie absorbante est directement obtenue par le procédé de l'invention.

Cette description est complétée par les dessins suivants:

## Breve description des figures

La figure 1 est une vue en coupe illustrant la structure du produit obtenu selon le mode de réalisation de l'invention.

La figure 2 représente en vue perspective le produit de la figure 1.

La figure 3 est un schéma illustrant les différentes étapes du mode de réalisation de l'invention.

La figure 4 est un schéma illustrant le dessin de la gravure des cylindres de calandrage.

La figure 5 illustre une variante du mode de réalisation avec dépôt de la poudre en plusieurs bandes.

La figure 6 est une coupe schématique d'une serviette périodique dont l'élément absorbant est obtenu selon le procédé de l'invention.

La figure 7 est une vue en perspective de la serviette périodique de la figure 7.

## Meilleure maniere de realiser l'invention

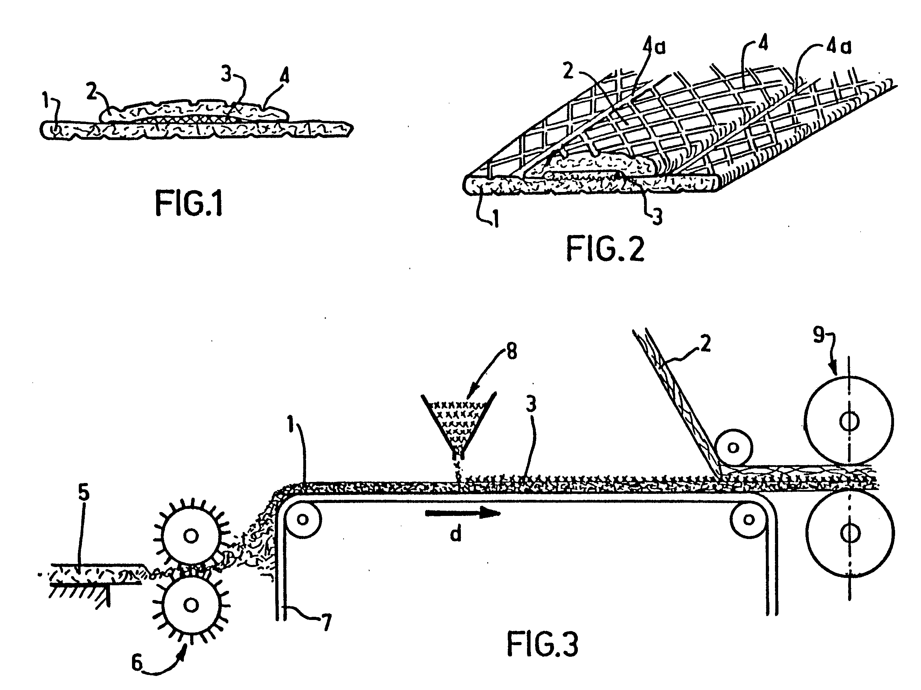

Selon le mode de réalisation de l'invention représenté sur le dessin des figures 1, 2 et 3, on dépose le produit super-absorbant en poudre 3 sur une nappe de mousse de cellulose 1 de faible épaisseur, comprise entre 0,5 mm et 5 mm, de préférence voisine de 1 mm.

Une telle nappe de mousse de cellulose est connue également sous le terme nappe de "fluff".

Il s'agit d'un matelas de fibres cellulosiques, obtenu par voie sèche, par une technique aujourd'hui parfaitement connue: simple défibrage à sec d'une feuille de pâte cellulosique, suivie d'une aspiration de fibres individuelles sur un support fixe ou un tapis transporteur. Le dépôt de la poudre super-absorbante peut être fait de manière continue avec une vitesse relativement grande pour permettre une productivité élevée.

Ensuite, une seconde nappe de fluff 2 de faible épaisseur également de préférence égale à celle de la nappe 1, recouvre le complexe ainsi obtenu. La poudre se trouve alors prise en "sandwich" entre les deux nappes de fluff 1 et 2.

Enfin, pour emprisonner la poudre super-absorbante et pour apporter une certaine cohésion aux nappes inférieure et supérieure, on procède au matriçage à froid de la structure fibreuse obtenue, en la faisant passer entre des rouleaux de matriçage portant sur la périphérie des dents linéaires qui formeront sur le produit des lignes de cohésion entre les deux nappes de fluff.

De façon préférentielle, on utilise des cylindres dont la gravure forme des alvéoles losangées.

A titre seulement d'exemple, les alvéoles peuvent être des losanges isocèles de 11 mm × 7 mm et de profondeur 1,5 mm.

Sur les figures 1 et 2, les lignes de matriçage sont schématisées et référencées 4.

On voit qu'elles forment un dessin réticulé, chaque maille emprisonnant une certaine quantité de poudre de superabsorbant.

Sur la figure 3, on a représenté par un schéma les différentes étapes de ce premier mode de réalisation du procédé.

La pâte 5 est défibrée à sec au moyen du défibreur référencé 6, les fibres cellulosiques sont ensuite aspirées par dépression sur un tapis transporteur se déplaçant selon la flèche d.

Sur la nappe 1 formée est déposée la poudre 3 au moyen d'un système d'alimentation et de dépôt schématisé en 8. La poudre est emprisonnée par recouvrement du complexe obtenu d'une seconde nappe de fluff 2.

L'ensemble est matricé à froid en 9 par passage dans les cylindres de gravure losangée.

La nappe de fluff présente une élasticité suffisante pour permettre une bonne expansion du gel formé après absorption.

Il est évident qu l'on peut réaliser de cette manière une structure fibreuse de longueur et de largeur quelconque, avec une ou plusieurs bandes de produit super-absorbant de largeur quelconque, le produit absorbant n'étant pas déposé dans le voisinage des bords latéraux, pour éviter des fuites de poudre.

En variante, on peut également, comme représenté sur le dessin de la figure 2, former deux lignes de matriçage 4a parallèles, le long des bords de la bande de produit super-absorbant.

Avec cette disposition, la poudre est enfermée de façon sûre dans la zone de dépôt.

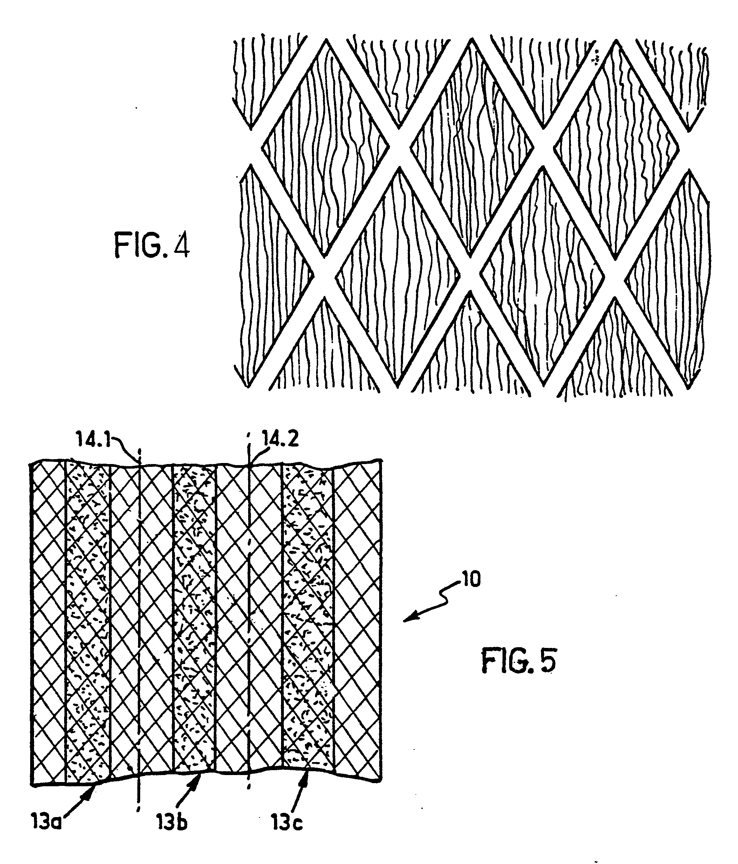

Selon une variante de réalisation du procédé illustré sur a figure 4 on dépose la poudre de produit super-absorbant en bandes sur une nappe de fibres dites super-frisées, puis on recouvre l'ensemble d'un deuxième matelas fibreux de fibres synthétiques de polyester et de polypropylène, également super-frisées.

Par rapport au mode de réalisation, cette variante présente l'avantage de fournir une structure fibreuse de présentation très intéressante.

En effet, en se reportant au dessin de la figure 5, dans laquelle on a représenté un dépôt de la poudre selon trois bandes 13a, 13b et 13c, il est aisé de découper ensuite selon les lignes de découpe 14.1 et 14.2, situées dans les zones sans poudre, pour obtenir des bandes de produit super-absorbant à partir de la structure 10 formée selon le procédé ci-dessus.

Les bandes sont ensuites enroulées pour offrir une présentation extrêmement pratique du produit.

La structure fibreuse obtenue de cette manière est très facile à utiliser puisqu'elle se présente en bobines dont la laize varie en fonction du produit fini à réaliser.

Quant à la fixation même du super-absorbant, elle offre les meilleurs avantages: le super-absorbant joue pleinement son rôle; le grain étant libre, dans une alvéole expansible (fibres super-frisées).

Le liquide à absorber est véhiculé et maintenu dans la bande super absorbante, du fait de la structure du complexe.

Les structures fibreuses comportant le produit super-absorbant, réalisées selon le procédé procédé décrit plus haut, sont avantageusement utilisées dans la réalisation de serviettes périodiques.

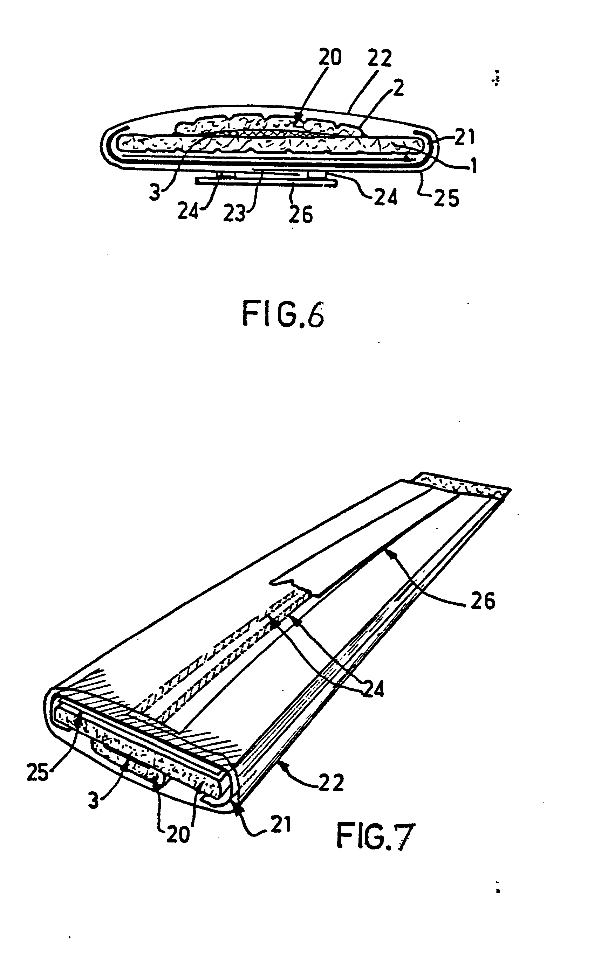

Dans les figures 6 et 7, on a représenté un exemple d'un serviette périodique constituée à partir de la structure fibreuse ainsi obtenue.

La serviette périodique se compose, de manière connue en soi, d'un noyau absorbant 20, obtenu selon le procédé décrit ci-dessus, la face non dirigée vers la peau de l'utilisatrice est recouverte d'un pli de polyéthylène ou similaire, référencée 21;

Ce pli, dit pli en U recouvre également les bords du noyau absorbant pour éviter les fuites latérales.

L'ensemble est enveloppé dans un pli de non-tissé 22 qui peut être fermé par un trait de colle 23.

Sur le côté de la serviette périodique, recouvert de la feuille de polyéthylène, la partie médiane est recouverte d'un ou plusieurs traits de colle 24 (adhésif "hot-melt", par exemple) pour la fixation de la serviette périodique.

L'adhésif est protégé, avant usage, par une bande de papier siliconé connue en soi, référencée 26.

Pour une serviette périodique le longueur 230 mm et de largeur 65 mm, on utilise deux nappes de fluff d'un poids total de 5 grammes et une quantité de produit super-absorbant en poudre comprise entre 0,5 et 3 grammes. Au noyau absorbant 20 peut éventuellement être associé une feuille de soutien 25 en tissue-ouate, pour maintien du "fluff".

**Revendications**

1. Procédé d'insertion de produits "super-absorbants" telsque des ethers cellulosiques, des alginates ou des polyacrylates modifiés, en poudre dans une structure fibreuse, consistant dans les étapes suivantes prises en combinaison:

a) on dépose, en au moins une bande dont la largeur est fonction du produit à réaliser, la poudre 3 de produits super-absorbant sur un matelas fibreux 1;

b) on recouvre l'ensemble d'une nappe de fibres 2,

caractérisé en ce qu'on lie uniquement par matriçage à froid la nappe de fibres 2 au dit matelas fibreux 1 selon des lignes et/ou des points de cohésion, de manière à définir entre ledit matelas 1 et ladite nappe 2 des espaces, dans lesquels une certaine quantité de poudre de produit super-absorbant est emprisonnée, les grains de poudre etant ainsi libres dans les espaces expansibles ainsi constitués.

2. Procédé selon la revendication 1, caractérisé en ce que le matriçage à froid est effectué selon un dessin réticulé 4, chaque maille enfermant une certaine quantité de poudre de produit super-absorbant.

3. Procédé selon l'une des revendications 1 ou 2, caractérisé en ce que le matriçage à froid s'effectue au moyen de cylindres de gravure, les alvéoles formées par ladite gravure étant losangées.

4. Procédé selon la revendication 3, caractérisé en ce que lesdites alvéoles sont des losanges isocèles de 11 mm×7 mm.

5. Procédé selon la revendication 1, caractérisé en ce que ladite nappe de fibres 2 est d'épaisseur comprise entre 0,5 mm et 5 mm, de préférence voisine de 1 mm.

6. Procédé selon la revendication 1, caractérisé en ce que ledit matelas fibreux 1 est également de faible épaisseur, de préférence égale à la nappe de fibres 2.

7. Procédé selon la revendication 1, caractérisé en ce que ladite nappe de fibres 2 est de largeur inférieure à la largeur du matelas 1, de manière à ne constituer qu'une nappe de recouvrement de la bande de produit super-absorbant.

8. Procédé d'obtention d'une structure fibreuse selon la revendication 1, renfermant un produit super-absorbant en poudre, consistant dans les étapes suivantes prises en combinaison:

— sur un matelas fibreux 1 de grande largeur, on dépose en plusieurs bandes parallèles la poudre 13 de produit super-absorbant,
— on recouvre le complexe obtenu d'une nappe fibreuse 2, au moins dans les zones où la poudre 13 est déposée,

caractérisé en ce qu'on

— lie par matriçage à froid la nappe fibreuse 2 audit matelas 1, selon des lignes et/ou des points de cohésion pour emprisonner la poudre de produit super-absorbant, de manière à définir entre ledit matelas 1 et ladite nappe 2 des espaces expansibles dans lesquels les grains de poudre se trouvent libres.
— découpe la structure 10 ainsi obtenu en bandes 13a, 13b, 13c selon des lignes de découpe 14 qui partagent longitudinalement les zones non recouvertes de poudre,
— enroule lesdites bandes ainsi obtenues en bobines.

9. Serviette périodique caractérisée par la structure suivante:

— un noyau absorbant 20, obtenu selon l'une quelconque des revendications 1 à 8, disposé éventuellement sur une feuille de soutien en tissue (25),
— un pli de polyéthylène 21 en U, recouvrant la face dudit noyau absorbant non dirigée vers la peau de l'utilisatrice et recouvrant les bords latéraux du noyau pour empécher les fuites latérales,
— une enveloppe 22 en non-tissé, entourant complètement le noyau et son pli de polyéthylène,
— une ou plusieurs bandes d'un produit adhésif 24, disposé longitudinalement dans la partie médiane de la face de la serviette non dirigée vers la peau de l'utilisatrice,
— une bande de papier siliconé 26 protégeant la ou lesdites bandes d'adhésif.

## Patentansprüche

1. Verfahren zum Einfügen von "superabsorbierenden" Produkten, wie Zelluloseether, Alginate oder modifizierte Polyacrylate, in Pulverform in eine Faserstruktur, mit folgender Stufenkombination

a) Aufbringen von mindestens einem Streifen des Pulvers (3) von superabsorbierenden Produkten in einer vom herzustellenden Produkt abhängenden Breite auf ein Faserpolster (1);
b) Bedecken des Ganzen mit einem Faservlies (2),

dadurch gekennzeichnet, daß man bloss durch kaltprägen das Faservlies (2) mit dem Faserpolster (1) in Übereinstimmung mit Kohäsions-Linien und/oder Punkten verbindet, so daß zwischen dem Faserpolster (1) und dem Faservlies (2) Räume abgegrenzt werden, in welchen eine bestimmte Menge des Pulvers aus superabsorbierendem Produkt eingeschlossen ist, wobei die Pulverkörner in den so gebildeten dehnbaren Räumen frei sind.

2. Verfahren nach Anspruch 1, dadurch gekennzeichnet, daß man das Kaltprägen in Übereinstimmung mit einem Netzmuster (4) bewirkt, von dem jede Masche eine bestimmte Menge des superabsorbierenden Pulvers einschließt.

3. Verfahren nach Anspruch 1 oder 2, dadurch gekennzeichnet, daß man das Kaltprägen mit Gravierwalzen vornimmt, wobei die Zellen, die durch die Gravur gebildet werden, rautenförmig sind.

4. Verfahren nach Anspruch 3, dadurch gekennzeichnet, daß die Zellen gleichschenkliger Rauten einer Größe von 11 mm×7 mm sind.

5. Verfahren nach Anspruch 1, dadurch gekennzeichnet, daß man ein Faservlies (2) einer Dicke von 0,5 bis 5 mm, vorzugsweise etwa 1 mm verwendet.

6. Verfahren nach Anspruch 1, dadurch gekennzeichnet, daß man ein Faserpolster (1) verwendet, das durchgehend von geringer Dicke, vorzugsweise von gleicher Dicke wie das Faservlies (2) ist.

7. Verfahren nach Anspruch 1, dadurch gekennzeichnet, daß man ein Faservlies (2) verwendet, das von geringerer Breite als das Faserpolster (1) ist, derart, daß es nur ein Vlies zum Bedecken des superabsorbierenden Streifens bildet.

8. Verfahren zur Herstellung einer ein superabsorbierendes Produkt in Pulverform einschließenden Faserstucktur nach Anspruch 1 mit folgenden Stufen:

Auflegen von mehreren parallelen Streifen des superabsorbieenden Pulvers (13) auf ein Faserpolster (1) großer Breite; und
Bedecken der erhaltenen Struktur mit einem Faservlies (2) mindestens in den Bereichen, in denen das Pulver (13) aufgelegt ist, dadurch gekennzeichnet, daß man das Faservlies (2) durch Kaltprägen mit dem Faserpolster (1) an Kohäsions-Linien und/oder -Punkten unter Einschluß des superabsorbierenden Pulvers (13) verbindet derart, daß zwischen dem Faserpolster (1) und dem Faservlies (2) dehnbare Räume begrenzt werden, in denen sich die Pulverkörner frei bewegen können;
die so erhaltene Struktur (10) zu Streifen (13a, 13b, 13c) entlang der Schneidlinien (14), welche der Länge nach über die nicht mit dem Pulver (13) bedeckten Bereiche verteilt sind, schneidet; und
die so erhaltenen Streifen auf Spulen aufwickelt.

9. Damenbinde, gekennzeichnet durch die folgende Struktur ein absorbierender Kern (20), erhalten nach einem der Ansprüche 1 bis 8, gegebenenfalls auf einer textilen Trägerfolie (25) angeordnet;

eine U-förmige Schicht (21) aus Polyethylen, welche die Vorderseite des gegen die Haut der Trägerin nicht gerichteten Kerns (20) abdeckt und die Seitenränder des Kerns (20) abdeckt, um seitliches Herausfließen zu verhindern;
eine Hülle (22) aus nicht gewebtem Stoff, die den Kern (20) und seine Polyethylenschicht vollkommen umhüllt;
ein oder mehrere Streifen eines Klebers (24), aufgebracht in Längsrichtung in dem Mittelteil der Vorderseite der gegen die Haut der Trägerin nicht gerichteten Binde;
und ein silikonierter Papierstreifen (26), der den oder die Klebstreifen schützt.

## Claims

1. Process for the insertion of "superabsorbent" products, such as cellulose ethers, alginatess or modified polyacrylates, in powder form, into a fibrous structure, which consists of the following steps taken in combination:

a) the powder 3 of superabsorbent products is deposited on a fibrous layer 1, in at least one strip whose width depends on the product to be made; and

b) the whole is covered with a sheet of fibres 2,

characterised in that the sheet of fibres 2 is bonded to the said fibrous layer 1, solely by cold embossing, along lines and/or points of cohesion, so as to define, between the said layer 1 and the said sheet 2, spaces in which a certain amount of powder of a superabsorbent product is trapped, the grains of powder thus being free in the expandable spaces formed in this way.

2. Process according to Claim 1, characterised in that the cold embossing is carried out according to a reticulated pattern 4, each mesh enclosing a certain amount of powder of superabsorbent product.

3. Process according to one of Claims 1 and 2, characterised in that the cold embossing is carried out by means of printing rollers, the cells formed by the said printing being diamond-shaped.

4. Process according to Claim 3, characterised in that the said cells are isosceles diamonds of 11 mm×7 mm.

5. Process according to Claim 1, characterised in that the said sheet of fibres 2 has a thickness of between 0.5 mm and 5 mm, preferably of the order of 1 mm.

6. Process according to Claim 1, characterised in that the said fibrous layer 1 is also of low thickness, preferably equal to that of the sheet of fibres 2.

7. Process according to Claim 1, characterised in that the width of the said sheet of fibres 2 is less than that of the layer 1, so as to form only one sheet covering the strip of superabsorbent product.

8. Process for obtaining a fibrous structure, according to Claim 1, containing a superabsorbent product in powder form, which consists of the following steps taken in combination:

— the powder 13 of superabsorbent product is deposited on a very wide, fibrous layer 1, in several parallel strips, and

— the composite structure obtained is covered with a fibrous sheet 2, at least in the areas where the powder 13 is deposited,

characterised in that

— the fibrous sheet 2 is bonded to the layer 1, by cold embossing, along lines and/or points of cohesion, in order to trap the powder of superabsorbent product, so as to define, between the said layer 1 and the said sheet 2, expandable spaces in which the grains of powder are free,

— the structure 10 thus obtained is cut into strips 13a, 13b, 13c along cutting lines 14, which longitudinally separate the areas not covered with powder, and

— the said strips thus obtained are wound into reels.

9. Sanitary towel, characterised by the following structure:

— an absorbent core 20 obtained according to any one of claims 1 to 8 and, if appropriate, placed on a backing sheet of fabric (25),

— a U-shaped ply of polyethylene 21 covering that face of the said absorbent core which is not directed towards the user's skin, and covering the lateral edges of the core in order to prevent lateral leaks,

— an envelope 22 made of nonwoven, completely surrounding the core and its polyethylene ply,

— one or more strips of an adhesive product 24 arranged longitudinally in the middle part of that face of the towel which is not directed towards the user's skin, and

— a strip of silicone-treated paper 26 protecting the said strip or strips of adhesive.

FIG.1

FIG.2

FIG.3

0 022 792

FIG. 4

FIG. 5

FIG.6

FIG.7